Europäisches Patentamt

European Patent Office

Office européen des brevets

(11) Publication number: **0 421 284 A1**

## (12) EUROPEAN PATENT APPLICATION

(21) Application number: 90118627.0

(22) Date of filing: 28.09.90

(51) Int. Cl.5: **C12N 15/16**, C12P 21/02, C07K 13/00, C12N 1/21, C12N 5/10, //(C12N1/21, C12R1:19)

The applicant has filed a statement in accordance with Rule 28 (4) EPC (issue of a sample only to an expert). Accession number(s) of the deposit(s): FERM BP-2617.

The applicant has subsequently filed a sequence listing and declared, that it includes no new matter.

(30) Priority: 30.09.89 JP 253797/89
27.10.89 JP 278497/89
02.07.90 JP 172735/90

(43) Date of publication of application:
10.04.91 Bulletin 91/15

(84) Designated Contracting States:
AT BE CH DE DK ES FR GB GR IT LI LU NL SE

(71) Applicant: TAKEDA CHEMICAL INDUSTRIES, LTD.
3-6, Doshomachi 2-chome Chuo-ku
Osaka 541(JP)

(72) Inventor: Onda, Haruo
5-26, Shimotakatsu 4-chome
Tsuchiura, Ibaraki 300(JP)
Inventor: Ohkubo, Shoichi
14-13, Takezono 2-chome
Tsukuba, Ibaraki 305(JP)

(74) Representative: von Kreisler, Alek, Dipl.-Chem. et al
Patentanwälte Von Kreisler-Selting-Werner,
Deichmannhaus am Hauptbahnhof
W-5000 Köln 1(DE)

(54) Human endothelin-3 cDNA and use thereof.

(57) A DNA comprising a cDNA coding for human endothelin-3 is disclosed. A precursor protein of human endothelin-3, a transformant transformed with the cDNA coding for human endothelin-3 and a method for producing endothelin-3 are also disclosed.

EP 0 421 284 A1

## HUMAN ENDOTHELIN-3 CDNA AND USE THEREOF

BACKGROUND OF THE INVENTION

The present invention relates to a DNA comprising a cDNA coding for human endothelin-3, to a precursor protein of human endothelin-3 and to a method for preparing endothelin-3.

In this specification, the term "precursor protein" includes a protein which includes an amino acid sequence of a mature peptide and has all or a portion of an amino acid sequence coded with a DNA of the peptide at the N-terminus, the C-terminus or both termini thereof.

There have been reports of endothelium-dependent vasoconstrictor reactions to various mechanical and chemical stimuli as well as endothelium-dependent vasodilative reactions. For example, it is known that vasoconstriction can be induced by mechanical loads such as vascular stretch and increased vascular inner pressure, or can be chemically induced by such agents as thrombin. Further, vasoconstriction can be induced by conditions of anoxia. Noradrenaline-induced vasoconstriction can be enhanced by use of neuropeptide Y [K. Takemoto, Proc. Natl. Acad. Sci. U.S.A. 79 , 5485 (1982); C. Minth et al., ibid. 81 , 4577 (1984)]. Endothelial cell-derived coronary vascular constrictor factors (each having molecular weights of 8,500 and 3,000) are described in K. A. Hickey et al., Am. J. Physiol. 248, C550(1985); and in R. F. O'Brien, J. Cell Physiol. 132 , 263 (1987). However, their structures are unknown. An endothelial cell-derived peptide-like substance is also described in M. N. Gillespie et al., J. Pharmac. Exp. Ther. 236 , 339 (1985). However, the structure of that substance is also unknown.

Vasopressin is known as a peptide having a vasoconstrictor activity, and the amino acid sequence thereof was determined. There have been no reports, however, that vasopressin was obtained from mammalian or bird vascular endothelial cells. Although there is a report that an angiotensin having a vasoconstrictor activity was obtained from the endothelial cells of bovine aortas [I. Kifor and V. J. Dzav, Circ. Res. 60 , 422 (1987)], the angiotensin is a peptide having a molecular weight of only about 1,000.

Some of the present inventors have previously succeeded in isolating porcine endothelin as a peptide having a similar vasoconstrictor activity from the endothelial cells of porcine aortas (Japanese Unexamined Patent Publication No. 206997/1989). Some of the present inventors have also succeeded in isolating human endothelin and cloning porcine endothelin cDNA and human endothelin cDNA (Japanese Patent Application Nos. 275613/1987, 313155/1987, 148158/1988 and 274454/1988). The mature polypeptides of the porcine endothelin and the human endothelin have the same amino acid sequence, and are referred to as endothelin-1.

Further, the present applicant has filed patent applications with respect to the isolation of rat endothelin and the cloning of its cDNA (Japanese Patent Application Nos. 174935/1988 and 188083/1988), and this rat endothelin is referred to as endothelin-3.

Furthermore, the present applicant has also filed a patent application with respect to the isolation of mouse endothelin and the cloning of its cDNA (Japanese Patent Application No. 223389/1988), and this mouse endothelin is referred to as endothelin B.

Further, the present applicant has succeeded in cloning a DNA which codes for a novel amino acid sequence of endothelin-2 from human genomic library (Japanese Patent Application No. 274990/1989).

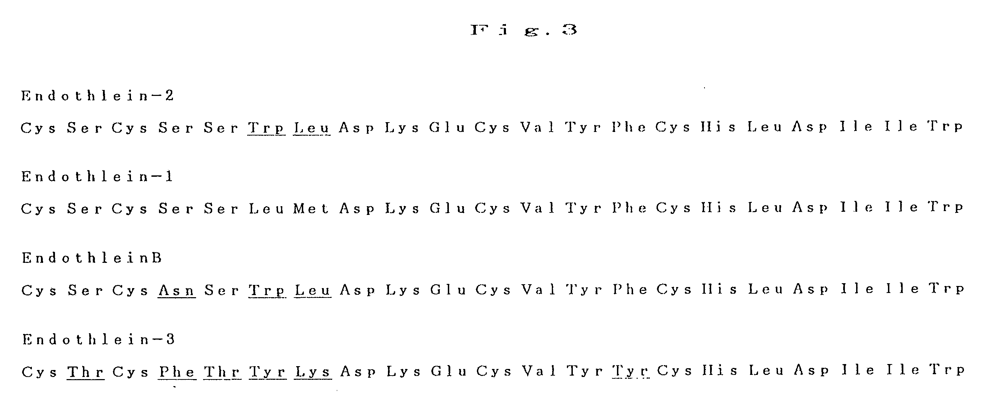

The amino acid sequences of the endothelin-1, endothelin-2, endothelin B and endothelin-3 are shown in Fig. 3 in comparison to one another.

Endothelin is a general term for peptides having a molecular weight of 2500 ± 300 and having 21 amino acid residues, including four cysteine groups located at the 1st, 3rd, 11th and 15th residues from the N-terminus of the amino acid sequence, which form two sets of disulfide bonds. One of the combinations of the disulfide bonds may be 1-15 and 3-11 cysteine groups, and the other may be 1-11 and 3-15. The former is higher in ratio of formation and in activity than the latter.

As described above, homologous endothelin peptides have been discovered from various animals. However, no novel homologous genes have been discovered from the same animal species. It is therefore a current subject that novel homologous endothelin is further screened, and the structure and activity of the endothelin is studied, thereby examining its usefulness, and that the novel peptide is cloned by gene recombination to pioneer mass production thereof.

SUMMARY OF THE INVENTION

Important contributions will be made to future studies and medical treatments, if a novel homologous gene having the vasoconstrictor activity described above can be collected and further prepared by gene recombination. As a result, the following information has been obtained, thus arriving at the present invention.

Namely, the present inventors have succeeded in cloning a complementary DNA (cDNA) coding for endothelin having an amino acid sequence different from that of the above endothelin-1[human endothelin (endothelin A)] from a human cDNA library by using as probes the synthesized DNA segment coding for a part of a genomic DNA of the human endothelin and a DNA with about 650 bp of a genomic DNA of endothelin-3 described in the patent applications previously filed. The present inventors have also succeeded in pioneering the mass production of the new endothelin by gene recombination. The present inventors have named this human endothelin having the novel amino acid sequence "human endothelin-3". The human endothelin 3 is the same as the above endothelin-3[rat endothelin (endothelin C)] in amino acid sequence of the mature protein, but different therefrom in nucleotide sequence coding for the mature protein and in preoursor amino acid sequence.

In accordance with the present invention, there are provided (1) a DNA comprising a cDNA coding for human endothelin-3, (2) a precursor protein of human endothelin-3, (3) a transformant containing a DNA comprising a cDNA coding for human endothelin-3 and (4) a method for preparing mature endothelin-3 which comprises culturing the transformant described in (3), producing and accumulating a protein in a culture medium, and collecting the protein thus obtained.

## BRIEF DESCRIPTION OF THE DRAWINGS

Fig. 1 shows a simplified restriction enzyme map of a DNA sequence comprising a human endothelin-3 precursor and a mature peptide;

Fig. 2 shows a nucleotide sequence of the human endothelin-3 precursor or mature peptide DNA, and the amino acid sequences deduced therefrom with mature endothelin-3 are boxed; and

Fig. 3 shows an amino acid sequence of the human endothelin-3 mature peptide deduced from the nucleotide sequence shown in Fig. 2, and amino acid sequences of endothelin-1, endothelin B and endothelin-2.

Fig. 4 shows a schematic representation of constructing expression plasmid pTS6009 for human endothelin-3;

Fig. 5 is a graph showing the change of endothelin-3 productivity with time of human endothelin-3 producing cell CHO-KI-15; and

Fig. 6 is an RP-HPLC diagram of immunoreactive endothelin produced by CHO-KI-15-4 cell.

## DESCRIPTION OF THE PREFERRED EMBODIMENTS

A cDNA of the present invention coding for human endothelin-3 comprises a nucleotide sequence corresponding to nucleotide sequence represented by the following formula (1) or a portion thereof, which is different from the known sequences and is novel.

```
                                          32
      CGAACCCCCACAGCTGGAGGGCGAGGCCAGCT

                                          77
GTACCCGGCCCCAGTGCCCTTTCGCGGCCACAAGCGGCCGTCCTC

                                         122
CTGGTCCGGTGCTCCGGCGCCTGATCTAGGTTCATGGAGCCGGGG

                                         167
CTGTGGCTCCTTTTCGGGCTCACAGTGACCTCCGCCGCAGGATTC

                                         212
GTGCCTTGCTCCCAGTCTGGGGATGCTGGCAGGCGCGGCGTGTCC

        Pst I    Bg l II                 257
CAGGCCCCCACTGCAGCCAGATCTGAGGGGGACTGTGAAGAGACT

                                         302
GTGGCTGGCCCTGGCGAGGAGACTGTGGCTGGCCCTGGCGAGGGG

                 Pst  I                   347
ACTGTGGCCCCGACAGCACTGCAGGGTCCAAGCCCTGGAAGCCCT

                 Apa  I                   392
GGGCAGGAGCAGGCGGCCGAGGGGGCCCCTGAGCACCACCGATCC

                                   AccI437
AGGCGCTGCACGTGCTTCACCTACAAGGACAAGGAGTGTGTCTAC

                                         482
TATTGCCACCTGGACATCATTTGGATCAACACTCCCGAACAGACG
```

```
                                   Hind III        527
GTGCCCTATGGACTGTCCAACTACAGAGGAAGCTTCCGGGGCAAG

                                    Pst   I        572
AGGTCTGCGGGGCCACTTCCAGGGAATCTGCAGCTCTCACATCGG

                                          617
CCACACTTGCGCTGCGCTTGTGTGGGGAGATATGACAAGGCCTGC

                                          662
CTGCACTTTTGCACCCAAACTCTGGACGTCAGCAGACAGGTTGAA

                                          707
GTCAAGGACCAACAAAGCAAGCAGGCTTTAGACCTCCACCATCCA

                                          752
AAGCTCATGCCCGGCAGTGGACTCGCCCTCGCTCCATCTACCTGC

                                          797
CCCCGCTGCCTCTTTCAGGAAGGAGCCCCTTAGGAGGACAGGCCT
                                         *
PstI                                      842
GCAGCATCCTGGTCTCGGGAGGCTTCTGTCATTGCTCACACACAG

                                          887
TTCAGATTTCCACCTCTTTATAGACAAGACGTGAATTTGCCTGGG   (1)
```

Human endothelin-3 precursor comprises the following amino acid sequence corresponding to amino acid sequence represented by the formula (2):

```
        M   E   P   G   L   W   L   L   F   G   L   T   V   T   S   15

        A   A   G   F   V   P   C   S   Q   S   G   D   A   G   R   30

        R   G   V   S   Q   A   P   T   A   A   R   S   E   G   D   45

        C   E   E   T   V   A   G   P   G   E   E   T   V   A   G   60

        P   G   E   G   T   V   A   P   T   A   L   Q   G   P   S   75

        P   G   S   P   G   Q   E   Q   A   A   E   G   A   P   E   90

        H   H   R   S   R   R   C   T   C   F   T   Y   K   D   K  105

        E   C   V   Y   Y   C   H   L   D   I   I   W   I   N   T  120

        P   E   Q   T   V   P   Y   G   L   S   N   Y   R   G   S  135

        F   R   G   K   R   S   A   G   P   L   P   G   B   L   Q  150
```

```
L  S  H  R  P  H  L  R  C  A  C  V  G  R  Y 165

D  K  A  C  L  H  F  C  T  Q  T  L  D  V  S 180

R  Q  V  E  V  K  D  Q  Q  S  K  Q  A  L  D 195

L  H  H  P  K  L  M  P  G  S  G  L  A  L  A 210

P  S  T  C  P  R  C  L  F  Q  E  G  A  P  * 224   (2).
```

The peptide having a following amino acid sequence (2′) of between the 97th and 138th positions in formula (2) is one of human endothelin-3 precursors and is named "big endothelin-3":

C T C F T Y K D K E C V Y Y C
H L D I I W I N T P E Q T V P
Y G L S N Y R G S F R G    (2′)

These human endothelin precursors are found for the first time and are novel.

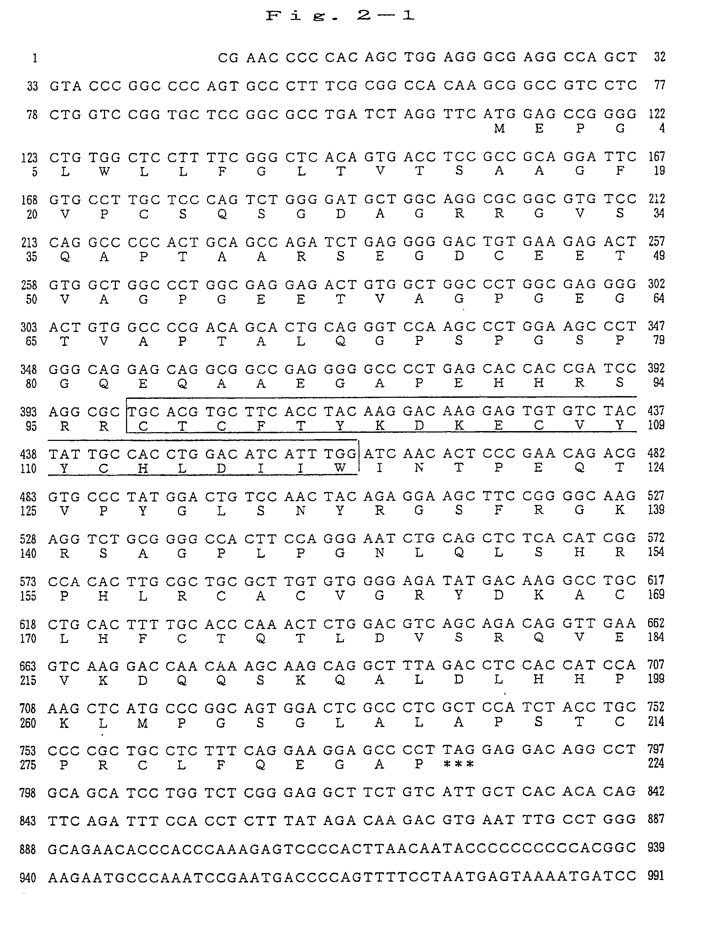

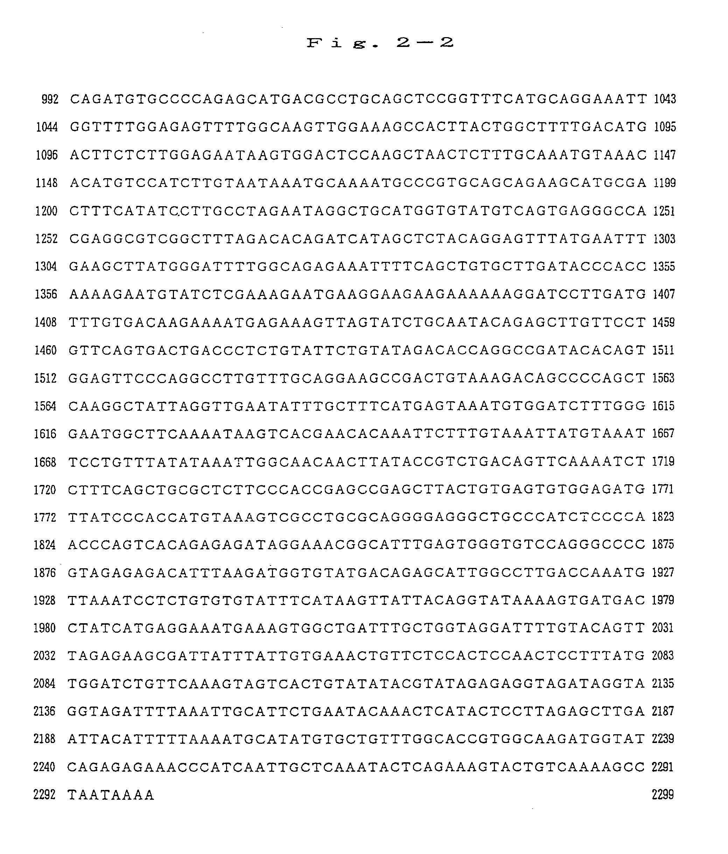

The amino acid sequence of mature endothelin-3 is boxed in Fig. 2 and is as follows:

C T C F T Y K D K E C V Y Y C
H L D I I W.

The nucleotide sequence coding for the above amino acid sequence may be, for example, one which is shown between 399th and 461st positions in formula (1).

In the present invention, for example, an expression vector having the DNA sequence containing the nucleotide sequence coding for mature endothelin-3 of human endothelin-3 can be prepared by the following process:

(a) Messenger RNA (mRNA) is isolated from human endothelin-3-producing cells.

(b) Single stranded complementary DNA (cDNA) is synthesized from the mRNA, followed by synthesis of double stranded DNA.

(c) The complementary DNA is introduced into a cloning vector such as a phage or a plasmid.

(d) Host cells are transformed with the recombinant phage or plasmid thus obtained.

(e) After cultivation of the transformants thus obtained, plasmids or phages containing the desired DNA are isolated from the transformants by an appropriate method such as hybridization with a DNA probe coding for a portion of human endothelin-3 or immunoassay using an anti-endothelin-3 antibody.

(f) The desired cloned DNA sequence is cut out from the recombinant DNA.

(g) The cloned DNA sequence or a portion thereof is ligated downstream from a promoter in the expression vector.

The mRNA coding for human endothelin-3 can be obtained from various endothelin-producing cells such as endothelial cells of human aortas and human placentas.

Methods for preparing the mRNA from the human endothelin-3-producing cells include the guanidine thiocyanate method [J. M. Chirgwin et al., Biochemistry 18 , 5294 (1979)] and the like.

Using the mRNA thus obtained as a template, cDNA is synthesized by use of reverse transcriptase, for example, in accordance with the method of H. Okayama et al. [Molecular and Cellular Biology 2 , 161 (1979); ibid. 3 , 280 (1983)]. The cDNA thus obtained is introduced into the plasmid.

The plasmids into which the cDNA may be introduced include, for example, pBR322 [Gene 2 , 95 (1977)], pBR325 [Gene 4 , 121 (1978)], pUC12 [Gene 19 , 259 (1982)] and pUC13 [Gene 19 , 259 (1982)], each derived from Escherichia coli , and pUB110 derived from Bacillus subtilis [Biochemical and Biophysical Research Communication 112 , 678 (1983)]. However, any other plasmid can be used as long as it is replicable and growable in the host cell. Examples of the phage vectors into which the cDNA may be introduced include λgtll [R. Young and R. Davis, Proc. Natl. Acad. Sci. U.S.A. 80 , 1194 (1983)]. However, any other phage vector can be used as long as it is growable in the host cell.

Methods for introducing the cDNA into the plasmid include, for example, the method described in T. Maniatis et al., Molecular Cloning , Cold Spring Harbor Laboratory, p.239 (1982). Methods for introducing the cDNA in the phage vector include, for example, the method of T. V. Hyunh et al. [DNA Cloning, A Practical Approach 1 , 49 (1985)].

The plasmid thus obtained is introduced into an appropriate host cell such as Esherichia and Bacillus .

Examples of Escherichia described above include Escherichia coli K12DHI [Proc. Natl. Acad. Sci. U.S.A. 60 , 160 (1968)], M103 [Nucleic Acids Research 9 , 309 (1981)], JA221 [Journal of Molecular Biology 120 , 517 (1978)] HB101 [Journal of Molecular Biology 41 , 459 (1969)] and C600 [Genetics 39 , 440 (1954)].

Examples of Bacillus described above include Bacillus subtilis MI114 [Gene 24 , 255 (1983)] and 207-21 [Journal of Biochemistry 95 , 87 (1984)].

Methods for transforming the host cell with the plasmid include, for example, the calcium chloride method or the calcium chloride/rubidium chloride method described in T. Maniatis et al., Molecular Cloning , Cold Spring Harbor Laboratory, p.249 (1982).

When a phage vector is used, for example, the phage vector can be transduced into multiplied Escherichia coli , using the in vitro packaging method.

Human cDNA libraries containing human endothelin-3 cDNA can be obtained by numerous techniques well known in the art including purchasing them from the market, though obtainable by the methods described above. For example, a cDNA library from human placentas is available from Clontech Laboratories, Inc., U.S.A.

Methods for cloning a human endothelin-3 cDNA from the human DNA library include, for example, the plaque hybridization method using oligonucleotides chemically synthesized on the basis of phage vector λcharon 4A and the amino acid sequence of human endothelin-3 as a probe [T. Maniatis et al., Molecular Cloning , Cold Spring Harbor Laboratory, (1982)]. The human endothelin-3 cDNA thus cloned is subcloned in, for example, pBR322, pUC12, pUC13, pUC19, pUC118 and pUC119 to obtain the human endothelin-3 DNA, if necessary.

The nucleotide sequence of the DNA sequence thus obtained is determined by, for example, the Maxam-Gilbert method [A. M. Maxam and W. Gilbert, Proc. Natl. Acad. Sci. U.S.A. 74 , 560 (1977)] or the dideoxy method [J. Messing et al., Nucleic Acids Research 9 , 309 (1981)], and the existence of the human endothelin-3 DNA is confirmed in comparison with the known amino acid sequence.

As described above, the DNA [human endothelin-3 cDNA, represented by formula (1)] coding for human endothelin-3 is obtained.

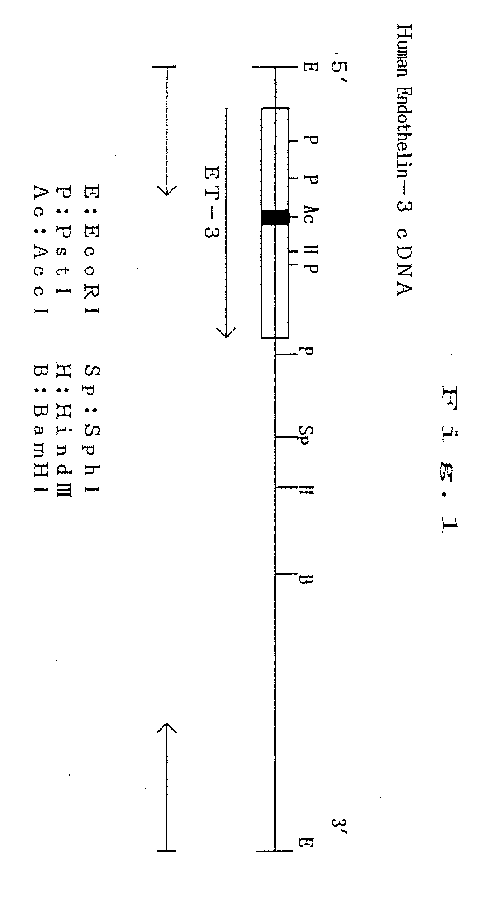

Fig. 1 shows a restriction enzyme fragment map of the cDNA coding for human endothelin-3 obtained in Example 2 described below. Fig. 2 shows the nucleotide sequence of the DNA as determined by the dideoxy method, and the amino acid sequence ascertained from the nucleotide sequence.

The cDNA coding for human endothelin-3 cloned as described above can be used as it is, or after digestion with a restriction enzyme if desired, depending on the intended use.

A region intended to be expressed is cut out from the cloned cDNA and ligated downstream from the promoter in a vehicle (vector) suitable for expression, whereby the expression vector can be obtained.

The DNA has ATG as a translation initiating codon at the 5′-terminus thereof and may have TAA, TGA or TAG as a translation terminating codon at the 3′-terminus. The translation initiating codon and translation terminating codon may be added by use of an appropriate synthetic DNA adaptor. A promoter is further ligated in the upstream thereof for the purpose of expressing the DNA sequence.

Examples of the vectors include the above plasmids derived from Escherichia coli such as pBR322, pBR325, pUC12, and pUC13, the plasmids derived from Bacillus subtilis such as pUB110, PTP5 and pC194, plasmids derived from yeast such as pSH19 and pSH15, bacteriophage such as λphage, and animal viruses such as retroviruses and vaccinia viruses.

As the promoter used in the present invention, any promoter is appropriate as long as the promoter is suitable for expression in the host cell selected for the gene expression.

When the host cell used for transformation is Escherichia , it is preferable that a trp promoter, a lac promoter, a recA promoter, a λPL promoter, a lpp promoter, etc. are used. When the host cell is Bacillus , it is preferable that a PHO5 promoter, a PGK promoter, a GAP promoter, an ADH promoter, etc. are used. In particular, it is preferable that the host cell is Escherichia and the promoter is the trp promoter or the λPL promoter.

When the host cell is an animal cell, a SV-40 derived promoter, a retrovirus promoter, a metallothionein promoter, a heat shock promoter, etc. are each usable.

An enhancer, a certain DNA sequence important for promoter's activity in a cell, is also effectively used for expression.

Using a vector containing the DNA sequence coding for the human endothelin-3 mature peptide thus constructed, transformants are prepared.

The host cells include, for example, Escherichia , Bacillus , yeast and animal cells.

As examples of the above Escherichia and Bacillus , strains similar to those described above can be mentioned.

Examples of the above yeast include Saccharomyces cerevisiae AH22, AH22R⁻, NA87-11A and DKD-5D.

Examples of the animal cells include monkey cell COS-7, Vero, Chinese hamster cell (CHO), mouse L cell and human FL cell.

The transformation of the above Escherichia is carried out according to, for example, the method described in Proc. Natl. Acad. Sci. U.S.A. 69 , 2110 (1972) or Gene 17 , 107 (1982).

The transformation of the above Bacillus is conducted according to, for example, the method described in Molecular & General Genetics 168 , 111 (1979).

The transformation of the yeast is carried out according to, for example, the method described in Proc. Natl. Acad. Sci. U.S.A. 75 , 1929 (1978).

The transformation of the animal cells is carried out according to, for example, the method described in Virology 52 , 456 (1973).

Thus, there are obtained transformants transformed with an expression vector containing the cDNA coding for the human endothelin-3 mature peptide.

When bacterial transformants are cultured, a liquid medium is particularly suitable as a medium used for culture. Carbon sources, nitrogen sources, inorganic compounds and others necessary for growth of the transformant are contained therein. Examples of the carbon sources include glucose, dextrin, soluble starch and sucrose. Examples of the nitrogen sources include inorganic or organic materials such as ammonium salts, nitrates, corn steep liquor, peptone, casein, meat extracts, soybean meal and potato extract solution. The inorganic compounds include, for example, calcium chloride, sodium dihydrogenphosphate and magnesium chloride. Yeast extract, vitamins, growth promoting factors and so on may be further added thereto.

The pH of the medium is preferably about 5 to 8. As the medium used for cultivation of Escherichia , there is preferred, for example, M9 medium containing glucose and Casamino Acids (Miller, Journal of Experiments in Molecular Genetics 431-433 , Cold Spring Harbor Laboratory, New York, 1972). In order to make the promoter act efficiently, a drug such as 3$\beta$-indolylacrylic acid may be added thereto if necessary.

When the host cell is Escherichia , the cultivation is usually carried out at about 15 to 43°C for about 3 to 24 hours, with aeration or agitation if necessary.

When the host cell is Bacillus , the cultivation is usually carried out at about 30 to 40°C for about 6 to 24 hours, with aeration or agitation if necessary.

When yeast transformants are cultured, there is used, for example, Burkholder minimum medium [K. L. Bostian et al., Proc. Natl. Acad. Sci. U.S.A. 77 , 4505 (1980)] as the medium. The pH of the medium is preferably adjusted to about 5 to 8. The cultivation is usually carried out at about 20 to 35°C for about 24 to 72 hours, with aeration or agitation if necessary.

When animal cell transformants are cultured, examples of the mediums include MEM medium containing about 5 to 20% fetal calf serum [Science 122 , 501 (1952)], DMEM medium [Virology 8 , 396 (1959)], RPMI1640 medium (Journal of the American Medical Association 199 , 519 (1967)] and 199 medium [Proceeding of the Society for the Biological Medicine 73 , 1 (1950). The pH is preferably about 6 to 8. The cultivation is usually carried out at about 30 to 40°C for about 15 to 60 hours, with aeration or agitation if necessary.

The human endothelin-3 mature peptide (endothelin-3) can be isolated and purified from the culture described above, for example, by the following method.

When the human endothelin-3 mature peptide is to be extracted from the cultured cells, the cells are collected by methods known in the art after cultivation. Then, the collected cells are suspended in an appropriate buffer solution and disrupted by ultrasonic treatment, lysozyme and/or freeze-thawing. Thereafter, a crude extracted solution of the human endothelin-3 mature peptide is obtained by centrifugation or filtration. The buffer solution may contain a protein denaturant such as urea or guanidine hydrochloride, or a surface-active agent such as Triton X-100.

When the human endothelin-3 precursor protein or mature peptide is secreted in the culture solution, a supernatant is separated from the cells by methods known in the art after the conclusion of cultivation, and then collected.

The separation and purification of the human endothelin-3 precursor protein or mature peptide contained in the culture supernatant or the extracted solution thus obtained can be performed by an appropriate combination of known separating and purifying methods. The known separating and purifying methods include methods utilizing solubility such as salt precipitation and solvent precipitation, methods mainly utilizing a difference in molecular weight such as dialysis, ultrafiltration, gel filtration and SDS-polyacrylamide gel electrophoresis, methods utilizing a difference in electric charge such as ion-exchange column chromatography, methods utilizing specific affinity such as affinity chromatography, methods utilizing a difference in hydrophobicity such as reverse phase high performance liquid chromatography and methods utilizing a difference in isoelectric point such as isoelectro-focusing electrophoresis.

The activity of the human endothelin-3 precursor protein or mature peptide thus formed can be measured by an enzyme immunoassay using a specific antibody. If the products have vasoconstrictive activity, this activity may also be measured as an index.

The cells, such as animal cells or Escherichia coli , transfected or transformed with the DNA sequence of the present invention allow large amounts of the endothelin-3 mature peptide to be produced. Hence, the production of these peptides can be advantageously achieved.

The endothelin-3 mature peptide prepared here not only can be utilized as hypotension therapeutic agents or local vasoconstrictors, but also give a clue to analysis of the mechanism of the vasoconstrictor reactions in vivo and to elucidation of antagonists to the vasoconstrictor factors, including the other endothelin peptides.

These peptides have such effects as preventing various kinds of hemorrhage, for example, gastric or esophageal hemorrhage as vasoconstrictors, and may also be useful in curing various shock symptoms. The peptides can be administered orally, locally, intravenously or parenterally, preferably locally or intravenously. The dose is 0.001 μg to 100 μg/kg, preferably 0.01 μg to 10 μg/kg. The dose is preferably dependent on weight and preferably used in the form of a solution in 1 to 10 ml of a saline solution.

The peptides of the present invention can be formed into various preparations together with additional components, such as emulsions, hydrated mixtures, tablets, solutions, powders, granules, capsules and pills. Examples of the additional components include pharmaceutically acceptable vehicles, disintegrators, lubricants, binders, dispersants, plasticizers, fillers and carriers. As to the additional components, examples of the vehicles include lactose, glucose and white sugar; those of the disintegrators include starch, sodium alginate, agar powder and carboxymethyl cellulose calcium; those of the lubricants include magnesium stearate, talc and liquid paraffin; those of the binders include syrup, gelatin solution, ethanol and polyvinyl alcohol; those of the dispersants include methyl cellulose, ethyl cellulose and shellac; and those of the plasticizers include glycerin and starch.

When nucleotides, amino acids and so on are indicated by the abbreviations in this specification and drawings, the abbreviations adopted by IUPAC-IUB Commission on Biochemical Nomenclature or commonly used in the art are employed. For example, the following abbreviations are used. When amino acids are capable of existing as optical isomers, the L-forms are represented unless otherwise specified.

DNA : Deoxyribonucleic acid
cDNA : Complementary deoxyribonucleic acid
A : Adenine
T : Thymine
G : Guanine
C : Cytosine
RNA : Ribonucleic acid
mRNA : Messenger ribonucleic acid
dATP : Deoxyadenosine triphosphate
dTTP : Deoxythymidine triphosphate
dGTP : Deoxyguanosine triphosphate
dCTP : Deoxycytidine triphosphate
ATP : Adenosine triphosphate
EDTA : Ethylenediaminetetraacetic acid
SDS : Sodium dodecyl sulfate
Gly or G : Glycine
Ala or A : Alanine
Val or V : Valine
Leu or L : Leucine
Ile or I : Isoleucine
Ser or S : Serine
Thr or T : Threonine
Cys of C : Cysteine
Met or M : Methionine
Glu or E : Glutamic acid
Asp or D : Aspartic acid
Lys or K : Lysine
Arg or R : Arginine
His or H : Histidine
Phe or F : Phenylalanine
Tyr or Y : Tyrosine
Trp or W : Tryptophan

Pro of P : Proline

Asn or N : Asparagine

Gln or Q : Glutamine

With respect to the human endothelin-3 of the present invention, a portion of the amino acid sequence may be modified, namely there may be addition, elimination or substitution with other amino acids.

The present invention will hereinafter be described in detail with the following Reference Example and Examples. It is understood that the Reference Example and Examples are not intended to limit the scope of the invention.

Transformant Escherichia coli DH5α/pHET-3(P) obtained in Example 2 was deposited with the Institute for Fermentation, Osaka, Japan (IFO) under the accession number IFO 14949 on September 25, 1989, and was deposited with the Fermentation Research Institute, Agency of Industrial Science and Technology, Ministry of International Trade and Industry, Japan (FRI) under the accession number FERM BP-2617 on September 29, 1989.

Reference Example

(1) Assay of Vascular Smooth Muscle Constrictor Activity

Porcine right coronary artery spiral specimens (0.5 X 20 mm) with the intima denuded by rubbing with a small swab are suspended in 3 ml of Krebs-Ringer solution maintained at 37°C and saturated with a mixed gas containing 5% carbon dioxide and 95% oxygen by volume. After setting the basal tension to 2 g, the isometric tension is measured with tension transducers.

(2) Assay of Cardiotonic Action

Instead of the porcine right coronary artery spiral specimens used in the assay described in the above item (1), suspended guinea pig right atrium specimens are used, and the tension and the heart rate per minute are measured according to the same procedure as described in (1).

Example 1

Preparation of DNA Probe Coding for a Portion of Genomic Human Endothelin-3

A DNA probe having the following sequence was chemically synthesized:

$5'$ GCG CCT GGA TCG GTG GTG CTC AGG GGC CCC$3'$

This sequence is a $5'$-side fragment of the human endothelin-3 DNA. The $5'$-terminus of a DNA with about 650 bp of cloned DNA of genomic human endothelin-3 was $^{32}$P-phosphorylated using T4 polynucleotide kinase. These probes were used for screening a genomic DNA library.

Example 2

Isolation of Human Endothelin-3 Precursor Genomic DNA and Determination of Nucleotide Sequence Thereof

Escherichia coli Y1090 was infected with the above human placenta-derived genomic DNA library (Clontech Laboratories, Inc.) and plated, whereby phage plaques were allowed to appear. According to the report of W. Benton and R. Davis [Science 196 , 180-182 (1977)], a portion of plaque DNA was transferred to a nylon filter and hybridized with the DNA probe labeled with $^{32}$P in Example 1. The hybridization was carried out in the presence of 20% formamide at 42°C, and then the filter was washed in 0.2 X SSC, 0.1% SDS at 20°C. Hybridization-positive clones were isolated. Then, a mature code region of gt10, one of these clones, was cut out with EcoRI and subcloned in plasmid pUC118. By transforming Escherichia coli DH5α with the resulting plasmid, transformant Escherichia coli DH5α/pHET-3(P) was obtained. There is shown in Fig. 1 the simplified restriction enzyme map of a human genomic DNA fragment contained in this plasmid. In the figure, the heavy bar

( ▮▮ )

shows a mature human endothelin-3 code region, and the following abbreviations are used E:EcoRI, P:PstI, Ac:AccI, Sp:SphI, H:HindIII, B:BamHI.

This mature peptide code region and the nucleotide sequence in the vicinity thereof were determined by the method of Sanger [Proc. Nat. Acad. Sci. U.S.A. 74 , 5463-5467 (1977)]. The nucleotide sequence and the amino acid sequence deduced therefrom are shown in Fig. 2. The region C to W framed by

[＿＿＿＿＿＿]

shows the mature peptide portion. The nucleotide number of the obtained cDNA is 2299 and it has a polyA signal AATAAA at the 3′-terminus. Fig. 3 shows, for comparison, the amino acid sequences of the mature peptides of endothelin-1, endothelin B and human endothelin-2 which have previously been discovered, along with the amino acid sequence of the human endothelin-3 mature peptide.

Example 3

Expression of Human Endothelin-3 in Animal Cell CHO-KI:

(1) Construction of Expression Plasmid:

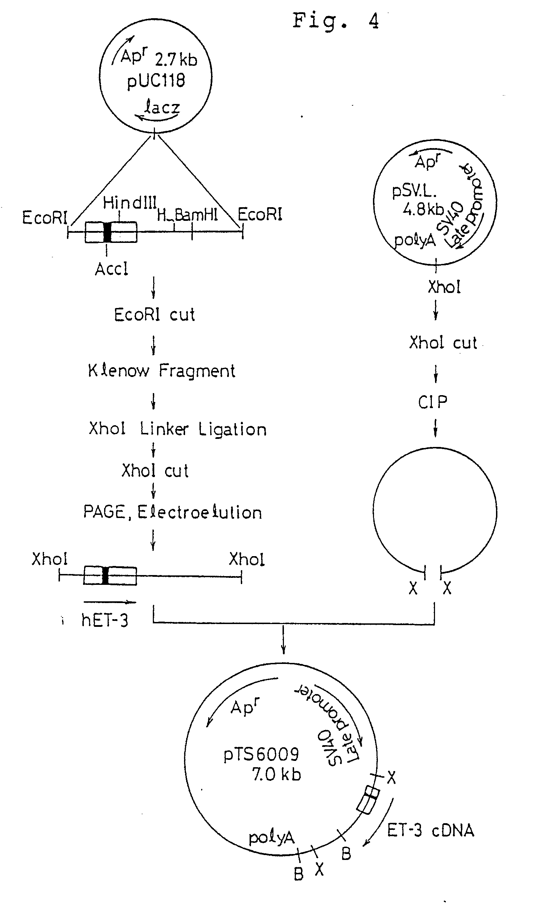

Purified plasmid pHET-3(P) containing cDNA coding for human endothelin-3 precursor and cloned from the above human placenta-derived cDNA library was treated with restriction enzyme EcoRI and the digested mixture was electrophoresed on 1% agarose gel to isolate a cDNA of 2.3 kb. The cDNA portion was electrically eluted and treated with Klenow fragment to convert the single strand portion at EcoRI digestion site into double stranded one. The resulting DNA was then ligated with a linker, 5′-pd-(CCTCGAGG)-3′, having a restriction enzyme XhoI-recognition sequence, followed by the treatment with XhoI. The resulting DNA was purified with PAGE(polyacrylamide gel electrophoresis) and electroeluted. The purified XhoI-ligated cDNA coding for a endothelin-3 precursor linked with the linker was incorporated into a XhoI site of expression vector pSVL to obtain plasmid pTS6009 (Fig. 4).

(2) Transformation of CHO-KI Cell:

CHO-KI cells were incubated in 6 cm falcon diameter plastic dishes containing Dulbecco's minimum essential medium (D-MEM) containing 10% bovine serum and supplemented with 100-50 μg/ml of streptomycin or kanamycin at 37° C with aeration of 5% $CO_2$, 95% air. When the cells proliferated by 80-90%, 1-10 μg of expression plasmid pTS6009 were applied to the cells in each dish according to the calcium phosphate method [Graham and Vandu Hba, Virology 52 , 456-467(1977)]. After about 7-18 hours, the cells were washed with -(Mg)PBS and the incubation was further continued in the above-mentioned medium.

CHO-KI cells were coinfected with plasmid pTS6009 and plasmid SV2-neo bearing a neomycin-resistant gene and incubated in a culture medium containing antibiotics G418. The G418-resistant cell lines were collected and named CHO-KI-15-4.

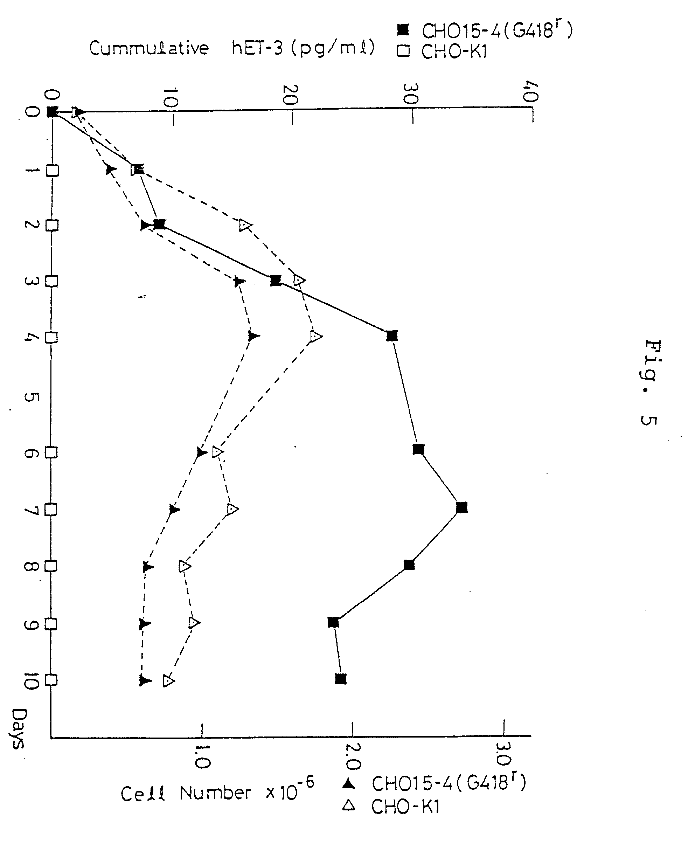

The CHO-KI-15-4 cell line was capable of stable expression of endothelin-3. As shown in Fig. 5, the measurement of the culture supernatant by enzyme immuno assay (EIA) specific to endothelin-3 reveals that the cell was able to continue the secretion of endothelin-3 for 3-8 days from the plant cf the cell. In Fig. 5, the left ordinate represents cumulative hET-3(pg/ml), while the right ordinate represents cell number (x $10^{-6}$). Plotted with ■ and ▲ are CHO-KI-15-4 (G418$^r$) while plotted with □ and △ are CHO-KI.

(3) Properties of Immunoreactive Endothelin-3 Produced by CHO-Kl-15-4 Cell:

A supernatant (about 70 ml) of a culture of CHO-Kl-15-4 cells was concentrated on Seppak C14 column. The concentrate (400 $\mu$l) was dissolved in 5% $CH_3CN$, 0.05% trifluoroacetic acid and subjected to RP-HPLC (reverse phase-high performance liquid chromatography) analysis.

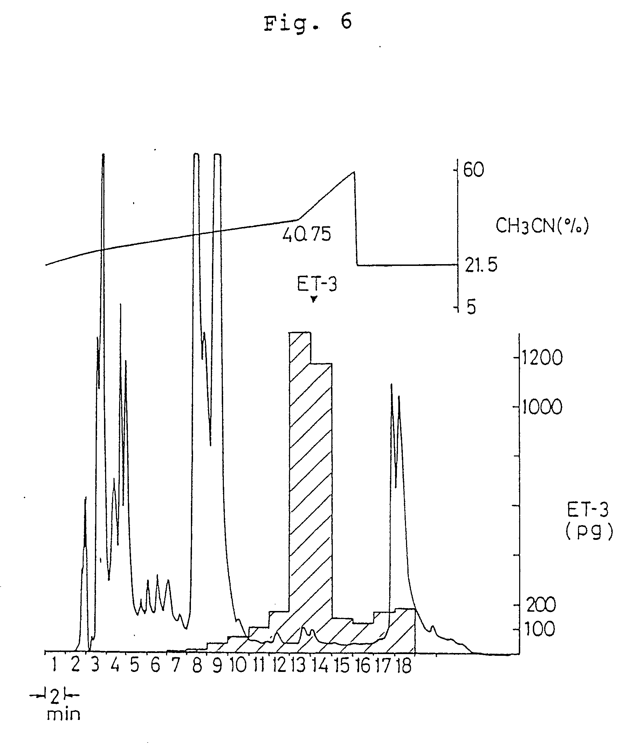

The fractionated samples were measured by EIA specific to endothelin-3. The results are shown in Fig. 6. Since EIA positive fraction was eluted in the same position as that of the synthesized endothelin-3, the immunoreaction positive endothelin secreted by CHO-K15-4 cells was considered to be endothelin-3 in view of the fact that the measurement was by sandwich type EIA specific to endothelin-3.

**Claims**

1. A DNA comprising a cDNA coding for human endothelin-3.
2. The DNA according to claim 1, wherein said cDNA comprises a nucleotide sequence corresponding to nucleotide sequence represented by the following formula (1) or a portion thereof:

```
                                              32
              CGAACCCCCACAGCTGGAGGGCGAGGCCAGCT

                                              77
         GTACCCGGCCCCAGTGCCCTTTCGCGGCCACAAGCGGCCGTCCTC

                                             122
         CTGGTCCGGTGCTCCGGCGCCTGATCTAGGTTCATGGAGCCGGGG

                                             167
         CTGTGGCTCCTTTTCGGGCTCACAGTGACCTCCGCCGCAGGATTC

                                             212
         GTGCCTTGCTCCCAGTCTGGGGATGCTGGCAGGCGCGGCGTGTCC

              Pst I    Bg l II                257
         CAGGCCCCCACTGCAGCCAGATCTGAGGGGGACTGTGAAGAGACT

                                             302
         GTGGCTGGCCCTGGCGAGGAGACTGTGGCTGGCCCTGGCGAGGGG

                   Pst   I                    347
         ACTGTGGCCCCGACAGCACTGCAGGGTCCAAGCCCTGGAAGCCCT

                      Apa   I                 392
         GGGCAGGAGCAGGCGGCCGAGGGGGCCCCTGAGCACCACCGATCC

                                       AccI437
         AGGCGCTGCACGTGCTTCACCTACAAGGACAAGGAGTGTGTCTAC

                                             482
         TATTGCCACCTGGACATCATTTGGATCAACACTCCCGAACAGACG

                                 Hind III     527
         GTGCCCTATGGACTGTCCAACTACAGAGGAAGCTTCCGGGGCAAG
```

```
                                 Pst  I              572
        AGGTCTGCGGGGCCACTTCCAGGGAATCTGCAGCTCTCACATCGG

                                                    617
        CCACACTTGCGCTGCGCTTGTGTGGGGAGATATGACAAGGCCTGC

                                                    662
        CTGCACTTTTGCACCCAAACTCTGGACGTCAGCAGACAGGTTGAA

                                                    707
        GTCAAGGACCAACAAAGCAAGCAGGCTTTAGACCTCCACCATCCA

                                                    752
        AAGCTCATGCCCGGCAGTGGACTCGCCCTCGCTCCATCTACCTGC

                                                    797
        CCCCGCTGCCTCTTTCAGGAAGGAGCCCCTTAGGAGGACAGGCCT
                                         *
        PstI                                        842
        GCAGCATCCTGGTCTCGGGAGGCTTCTGTCATTGCTCACACACAG

                                                    887
        TTCAGATTTCCACCTCTTTATAGACAAGACGTGAATTTGCCTGGG   (1).
```

3. A precursor protein of human endothelin-3.

4. The precursor protein according to claim 3, which comprises the following amino acid:

C T C F T Y K D K E C V Y Y C
H L D I I W I N T P E Q T V P
Y G L S N Y R G S F R G

5. The precursor protein according to claim 4, which comprises an amino acid sequence corresponding to amino acid sequence represented by the following formula (2):

```
    M   E   P   G   L   W   L   L   F   G   L   T   V   T   S   15

    A   A   G   F   V   P   C   S   Q   S   G   D   A   G   R   30

    R   G   V   S   Q   A   P   T   A   A   R   S   E   G   D   45
```

13

| | | | | | | | | | | | | | | | |
|---|---|---|---|---|---|---|---|---|---|---|---|---|---|---|---|
| C | E | E | T | V | A | G | P | G | E | E | T | V | A | G | 60 |
| P | G | E | G | T | V | A | P | T | A | L | Q | G | P | S | 75 |
| P | G | S | P | G | Q | E | Q | A | A | E | G | A | P | E | 90 |
| H | H | R | S | R | R | C | T | C | F | T | Y | K | D | K | 105 |
| E | C | V | Y | Y | C | H | L | D | I | I | W | I | N | T | 120 |
| P | E | Q | T | V | P | Y | G | L | S | N | Y | R | G | S | 135 |
| F | R | G | K | R | S | A | G | P | L | P | G | B | L | Q | 150 |
| L | S | H | R | P | H | L | R | C | A | C | V | G | R | Y | 165 |
| D | K | A | C | L | H | F | C | T | Q | T | L | D | V | S | 180 |
| R | Q | V | E | V | K | D | Q | Q | S | K | Q | A | L | D | 195 |
| L | H | H | P | K | L | M | P | G | S | G | L | A | L | A | 210 |
| P | S | T | C | P | R | C | L | F | Q | E | G | A | P | * | 224 (2). |

6. A transformant transformed with a DNA comprising a cDNA coding for human endothelin-3.

7. The transformant according to claim 6, which has the characteristics of E. coli DH 5α/pHET-3(P) (FERM BP-2617).

8. The transformant according to claim 6, which has the characteristics of CHO-KI-15-4.

9. A production method for a mature endothelin-3 protein which comprises (a) culturing the transformants according to claim 6, (b) accumulating mature endothelin-3 in a culture medium, and collecting the same.

SEQUENCE   NR. 1

```
  1                        CG AAC CCC CAC AGC TGG AGG GCG AGG CCA GCT   32

 33   GTA CCC GGC CCC AGT GCC CTT TCG CGG CCA CAA GCG GCC GTC CTC   77

 78   CTG GTC CGG TGC TCC GGC GCC TGA TCT AGG TTC ATG GAG CCG GGG  122
                                                        M   E   P   G    4

123   CTG TGG CTC CTT TTC GGG CTC ACA GTG ACC TCC GCC GCA GGA TTC  167
  5    L   W   L   L   F   G   L   T   V   T   S   A   A   G   F    19

168   GTG CCT TGC TCC CAG TCT GGG GAT GCT GGC AGG CGC GGC GTG TCC  212
 20    V   P   C   S   Q   S   G   D   A   G   R   R   G   V   S    34

213   CAG GCC CCC ACT GCA GCC AGA TCT GAG GGG GAC TGT GAA GAG ACT  257
 35    Q   A   P   T   A   A   R   S   E   G   D   C   E   E   T    49

258   GTG GCT GGC CCT GGC GAG GAG ACT GTG GCT GGC CCT GGC GAG GGG  302
 50    V   A   G   P   G   E   E   T   V   A   G   P   G   E   G    64

303   ACT GTG GCC CCG ACA GCA CTG CAG GGT CCA AGC CCT GGA AGC CCT  347
 65    T   V   A   P   T   A   L   Q   G   P   S   P   G   S   P    79

348   GGG CAG GAG CAG GCG GCC GAG GGG GCC CCT GAG CAC CAC CGA TCC  392
 80    G   Q   E   Q   A   A   E   G   A   P   E   H   H   R   S    94

393   AGG CGC TGC ACG TGC TTC ACC TAC AAG GAC AAG GAG TGT GTC TAC  437
 95    R   R   C   T   C   F   T   Y   K   D   K   E   C   V   Y   109

438   TAT TGC CAC CTG GAC ATC ATT TGG ATC AAC ACT CCC GAA CAG ACG  462
110    Y   C   H   L   D   I   I   W   I   N   T   P   E   Q   T   124

483   GTG CCC TAT GGA CTG TCC AAC TAC AGA GGA AGC TTC CGG GGC AAG  527
125    V   P   Y   G   L   S   N   Y   R   G   S   F   R   G   K   139

528   AGG TCT GCG GGG CCA CTT CCA GGG AAT CTG CAG CTC TCA CAT CGG  572
140    R   S   A   G   P   L   P   G   N   L   Q   L   S   H   R   154

573   CCA CAC TTG CGC TGC GCT TGT GTG GGG AGA TAT GAC AAG GCC TGC  617
155    P   H   L   R   C   A   C   V   G   R   Y   D   K   A   C   169

618   CTG CAC TTT TGC ACC CAA ACT CTG GAC GTC AGC AGA CAG GTT GAA  662
170    L   H   F   C   T   Q   T   L   D   V   S   R   Q   V   E   184

663   GTC AAG GAC CAA CAA AGC AAG CAG GCT TTA GAC CTC CAC CAT CCA  707
215    V   K   D   Q   Q   S   K   Q   A   L   D   L   H   H   P   199

708   AAG CTC ATG CCC GGC AGT GGA CTC GCC CTC GCT CCA TCT ACC TGC  752
260    K   L   M   P   G   S   G   L   A   L   A   P   S   T   C   214

753   CCC CGC TGC CTC TTT CAG GAA GGA GCC CCT TAG GAG GAC AGG CCT  797
275    P   R   C   L   F   Q   E   G   A   P   * * *               224

798   GCA GCA TCC TGG TCT CGG GAG GCT TCT GTC ATT GCT CAC ACA CAG  842

843   TTC AGA TTT CCA CCT CTT TAT AGA CAA GAC GTG AAT TTG CCT GGG  887

888   GCAGAACACCCACCCAAAGAGTCCCCACTTAACAATACCCCCCCCCCACGGC         939

940   AAGAATGCCCAAATCCGAATGACCCCAGTTTTCCTAATGAGTAAAATGATCC         991
```

```
CAGATGTGCCCCAGAGCATGACGCCTGCAGCTCCGGTTTCATGCAGGAAATT
GGTTTTGGAGAGTTTTGGCAAGTTGGAAAGCCACTTACTGGCTTTTGACATG
ACTTCTCTTGGAGAATAAGTGGACTCCAAGCTAACTCTTTGCAAATGTAAAC
ACATGTCCATCTTGTAATAAATGCAAAATGCCCGTGCAGCAGAAGCATGCGA
CTTTCATATCCTTGCCTAGAATAGGCTGCATGGTGTATGTCAGTGAGGGCCA
CGAGGCGTCGGCTTTAGACACAGATCATAGCTCTACAGGAGTTTATGAATTT
GAAGCTTATGGGATTTTGGCAGAGAAATTTTCAGCTGTGCTTGATACCCACC
AAAAGAATGTATCTCGAAAGAATGAAGGAAGAAGAAAAAAGGATCCTTGATG
TTTGTGACAAGAAAATGAGAAAGTTAGTATCTGCAATACAGAGCTTGTTCCT
GTTCAGTGACTGACCCTCTGTATTCTGTATAGACACCAGGCCGATACACAGT
GGAGTTCCCAGGCCTTGTTTGCAGGAAGCCGACTGTAAAGACAGCCCCAGCT
CAAGGCTATTAGGTTGAATATTTGCTTTCATGAGTAAATGTGGATCTTTGGG
GAATGGCTTCAAAATAAGTCACGAACACAAATTCTTTGTAAATTATGTAAAT
TCCTGTTTATATAAATTGGCAACAACTTATACCGTCTGACAGTTCAAAATCT
CTTTCAGCTGCGCTCTTCCCACCGAGCCGAGCTTACTGTGAGTGTGGAGATG
TTATCCCACCATGTAAAGTCGCCTGCGCAGGGGAGGGCTGCCCATCTCCCCA
ACCCAGTCACAGAGAGATAGGAAACGGCATTTGAGTGGGTGTCCAGGGCCCC
GTAGAGAGACATTTAAGATGGTGTATGACAGAGCATTGGCCTTGACCAAATG
TTAAATCCTCTGTGTGTATTTCATAAGTTATTACAGGTATAAAAGTGATGAC
CTATCATGAGGAAATGAAAGTGGCTGATTGCTGGTAGGATTTTGTACAGTT
TAGAGAAGCGATTATTTATTGTGAAACTGTTCTCCACTCCAACTCCTTTATG
TGGATCTGTTCAAAGTAGTCACTGTATATACGTATAGAGAGGTAGATAGGTA
GGTAGATTTTAAATTGCATTCTGAATACAAACTCATACTCCTTAGAGCTTGA
ATTACATTTTTAAAATGCATATGTGCTGTTTGGCACCGTGGCAAGATGGTAT
CAGAGAGAAACCCATCAATTGCTCAAATACTCAGAAGTACTGTCAAAGCC
TAATAAAA
```

16

( i )

Sequence type   :  nucleotide sequence

Sequence length:  2299 bases

Molecular type  :  DNA

Original Source:  human

Properties      :  human endothelin-3 complementary DNA

( ii )

Sequence type   :  nucleotide sequence

Sequence length:  887 bases [No. 1-887 of Formula II- (i) ]

Molecular type  :  DNA

Original Source:  human

Properties      :  human endothelin-3 complementary DNA

SEQUENCE   NR.  2

```
M   E   P   G   L   W   L   L   F   G   L   T   V   T   S  15

A   A   G   F   V   P   C   S   Q   S   G   D   A   G   R  30

R   G   V   S   Q   A   P   T   A   A   R   S   E   G   D  45

C   E   E   T   V   A   G   P   G   E   E   T   V   A   G  60

P   G   E   G   T   V   A   P   T   A   L   Q   G   P   S  75

P   G   S   P   G   Q   E   Q   A   A   E   G   A   P   E  90

H   H   R   S   R   R   C   T   C   F   T   Y   K   D   K 105

E   C   V   Y   Y   C   H   L   D   I   I   W   I   N   T 120

P   E   Q   T   V   P   Y   G   L   S   N   Y   R   G   S 135

F   R   G   K   R   S   A   G   P   L   P   G   B   L   Q 150

L   S   H   R   P   H   L   R   C   A   C   V   G   R   Y 165

D   K   A   C   L   H   F   C   T   Q   T   L   D   V   S 180

R   Q   V   E   V   K   D   Q   Q   S   K   Q   A   L   D 195

L   H   H   P   K   L   M   P   G   S   G   L   A   L   A 210

P   S   T   C   P   R   C   L   F   Q   E   G   A   P   * 224
```

Sequence type   :  polypeptide sequence
Sequence length:  224 amino acids
Molecular type  :  protein
Original Source:  human
Properties      :  human endothelin-3 precursor

## SEQUENCE NR. 3

```
C   T   C   F   T   Y   K   D   K   E   C   V   Y   Y   C

H   L   D   I   I   W   I   N   T   P   E   Q   T   V   P

Y   G   L   S   N   Y   R   G   S   F   R   G
```

Sequence type   :  polypeptide sequence
Sequence length:  42 amino acids [No. 97-138 of Formula II]
Molecular type :  protein
Original Source:  human
Properties      :  human endothelin-3 precusor(a "Big Endothelin-3")

EP 0 421 284 A1

# F i g . 1

Human Endothelin—3 cDNA

E : E c o R I    S p : S p h I
P : P s t I    H : H i n d III
A c : A c c I    B : B a m H I

## Fig. 2－1

```
  1                              CG AAC CCC CAC AGC TGG AGG GCG AGG CCA GCT   32

 33  GTA CCC GGC CCC AGT GCC CTT TCG CGG CCA CAA GCG GCC GTC CTC   77

 78  CTG GTC CGG TGC TCC GGC GCC TGA TCT AGG TTC ATG GAG CCG GGG  122
                                                      M   E   P   G    4

123  CTG TGG CTC CTT TTC GGG CTC ACA GTG ACC TCC GCC GCA GGA TTC  167
  5   L   W   L   L   F   G   L   T   V   T   S   A   A   G   F    19

168  GTG CCT TGC TCC CAG TCT GGG GAT GCT GGC AGG CGC GGC GTG TCC  212
 20   V   P   C   S   Q   S   G   D   A   G   R   R   G   V   S    34

213  CAG GCC CCC ACT GCA GCC AGA TCT GAG GGG GAC TGT GAA GAG ACT  257
 35   Q   A   P   T   A   A   R   S   E   G   D   C   E   E   T    49

258  GTG GCT GGC CCT GGC GAG GAG ACT GTG GCT GGC CCT GGC GAG GGG  302
 50   V   A   G   P   G   E   E   T   V   A   G   P   G   E   G    64

303  ACT GTG GCC CCG ACA GCA CTG CAG GGT CCA AGC CCT GGA AGC CCT  347
 65   T   V   A   P   T   A   L   Q   G   P   S   P   G   S   P    79

348  GGG CAG GAG CAG GCG GCC GAG GGG GCC CCT GAG CAC CAC CGA TCC  392
 80   G   Q   E   Q   A   A   E   G   A   P   E   H   H   R   S    94

393  AGG CGC TGC ACG TGC TTC ACC TAC AAG GAC AAG GAG TGT GTC TAC  437
 95   R   R   C   T   C   F   T   Y   K   D   K   E   C   V   Y   109

438  TAT TGC CAC CTG GAC ATC ATT TGG ATC AAC ACT CCC GAA CAG ACG  482
110   Y   C   H   L   D   I   I   W   I   N   T   P   E   Q   T   124

483  GTG CCC TAT GGA CTG TCC AAC TAC AGA GGA AGC TTC CGG GGC AAG  527
125   V   P   Y   G   L   S   N   Y   R   G   S   F   R   G   K   139

528  AGG TCT GCG GGG CCA CTT CCA GGG AAT CTG CAG CTC TCA CAT CGG  572
140   R   S   A   G   P   L   P   G   N   L   Q   L   S   H   R   154

573  CCA CAC TTG CGC TGC GCT TGT GTG GGG AGA TAT GAC AAG GCC TGC  617
155   P   H   L   R   C   A   C   V   G   R   Y   D   K   A   C   169

618  CTG CAC TTT TGC ACC CAA ACT CTG GAC GTC AGC AGA CAG GTT GAA  662
170   L   H   F   C   T   Q   T   L   D   V   S   R   Q   V   E   184

663  GTC AAG GAC CAA CAA AGC AAG CAG GCT TTA GAC CTC CAC CAT CCA  707
215   V   K   D   Q   Q   S   K   Q   A   L   D   L   H   H   P   199

708  AAG CTC ATG CCC GGC AGT GGA CTC GCC CTC GCT CCA TCT ACC TGC  752
260   K   L   M   P   G   S   G   L   A   L   A   P   S   T   C   214

753  CCC CGC TGC CTC TTT CAG GAA GGA GCC CCT TAG GAG GAC AGG CCT  797
275   P   R   C   L   F   Q   E   G   A   P   ＊＊＊                224

798  GCA GCA TCC TGG TCT CGG GAG GCT TCT GTC ATT GCT CAC ACA CAG  842

843  TTC AGA TTT CCA CCT CTT TAT AGA CAA GAC GTG AAT TTG CCT GGG  887

888  GCAGAACACCCACCCAAAGAGTCCCCACTTAACAATACCCCCCCCCCACGGC  939

940  AAGAATGCCCAAATCCGAATGACCCCAGTTTTCCTAATGAGTAAAATGATCC  991
```

## Fig. 2-2

```
992  CAGATGTGCCCCAGAGCATGACGCCTGCAGCTCCGGTTTCATGCAGGAAATT 1043
1044 GGTTTTGGAGAGTTTTGGCAAGTTGGAAAGCCACTTACTGGCTTTTGACATG 1095
1096 ACTTCTCTTGGAGAATAAGTGGACTCCAAGCTAACTCTTTGCAAATGTAAAC 1147
1148 ACATGTCCATCTTGTAATAAATGCAAAATGCCCGTGCAGCAGAAGCATGCGA 1199
1200 CTTTCATATCCTTGCCTAGAATAGGCTGCATGGTGTATGTCAGTGAGGGCCA 1251
1252 CGAGGCGTCGGCTTTAGACACAGATCATAGCTCTACAGGAGTTTATGAATTT 1303
1304 GAAGCTTATGGGATTTTGGCAGAGAAATTTTCAGCTGTGCTTGATACCCACC 1355
1356 AAAAGAATGTATCTCGAAAGAATGAAGGAAGAAGAAAAAGGATCCTTGATG 1407
1408 TTTGTGACAAGAAAATGAGAAAGTTAGTATCTGCAATACAGAGCTTGTTCCT 1459
1460 GTTCAGTGACTGACCCTCTGTATTCTGTATAGACACCAGGCCGATACACAGT 1511
1512 GGAGTTCCCAGGCCTTGTTTGCAGGAAGCCGACTGTAAAGACAGCCCCAGCT 1563
1564 CAAGGCTATTAGGTTGAATATTTGCTTTCATGAGTAAATGTGGATCTTTGGG 1615
1616 GAATGGCTTCAAAATAAGTCACGAACACAAATTCTTTGTAAATTATGTAAAT 1667
1668 TCCTGTTTATATAAATTGGCAACAACTTATACCGTCTGACAGTTCAAAATCT 1719
1720 CTTTCAGCTGCGCTCTTCCCACCGAGCCGAGCTTACTGTGAGTGTGGAGATG 1771
1772 TTATCCCACCATGTAAAGTCGCCTGCGCAGGGGAGGGCTGCCCATCTCCCCA 1823
1824 ACCCAGTCACAGAGAGATAGGAAACGGCATTTGAGTGGGTGTCCAGGGCCCC 1875
1876 GTAGAGAGACATTTAAGATGGTGTATGACAGAGCATTGGCCTTGACCAAATG 1927
1928 TTAAATCCTCTGTGTGTATTTCATAAGTTATTACAGGTATAAAAGTGATGAC 1979
1980 CTATCATGAGGAAATGAAAGTGGCTGATTGCTGGTAGGATTTTGTACAGTT 2031
2032 TAGAGAAGCGATTATTTATTGTGAAACTGTTCTCCACTCCAACTCCTTTATG 2083
2084 TGGATCTGTTCAAAGTAGTCACTGTATATACGTATAGAGAGGTAGATAGGTA 2135
2136 GGTAGATTTTAAATTGCATTCTGAATACAAACTCATACTCCTTAGAGCTTGA 2187
2188 ATTACATTTTTAAAATGCATATGTGCTGTTTGGCACCGTGGCAAGATGGTAT 2239
2240 CAGAGAGAAACCCATCAATTGCTCAAATACTCAGAAAGTACTGTCAAAAGCC 2291
2292 TAATAAAA                                               2299
```

# Fig. 3

Endothlein-2

Cys Ser Cys Ser Ser Trp Leu Asp Lys Glu Cys Val Tyr Phe Cys His Leu Asp Ile Ile Trp

Endothlein-1

Cys Ser Cys Ser Ser Leu Met Asp Lys Glu Cys Val Tyr Phe Cys His Leu Asp Ile Ile Trp

EndothleinB

Cys Ser Cys Asn Ser Trp Leu Asp Lys Glu Cys Val Tyr Phe Cys His Leu Asp Ile Ile Trp

Endothlein-3

Cys Thr Cys Phe Thr Tyr Lys Asp Lys Glu Cys Val Tyr Tyr Cys His Leu Asp Ile Ile Trp

Fig. 4

Fig. 5

# Fig. 6

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | PROCEEDINGS OF THE NATIONAL ACADEMY OF SCIENCES OF USA. vol. 86, April 1989, WASHINGTON US pages 2863 - 2867; INOUE A. et al: "The human endothelin family:3 structurally and pharmacologically distinct isopeptides predicted by 3 separate genes" * the whole document * | 1-6 | C12N15/16 C12P21/02 C07K13/00 C12N1/21.// (C12N1/21, C12R1:19) C12N5/10 |
| X | PROCEEDINGS OF THE NATIONAL ACADEMY OF SCIENCES OF USA. vol. 85, September 1988, WASHINGTON US pages 6964 - 6967; YANAGISAWA M. et al: "Primary structure,synthesis,and biological activity of rat endothelin, anendothelium-derived vasoconstrictor peptide" * the whole document * | 3, 4 | |
| P,X | FEBS LETTERS. vol. 261, no. 2, February 1990, AMSTERDAM NL pages 327 - 330; ONDA H. et al: "One of the endothelin gene family,endothelin 3 gene,is expressed in the placenta" * the whole document * | 1-7, 9 | |
| | | | TECHNICAL FIELDS SEARCHED (Int. Cl.5 ) |
| P,X | EP-A-366016 (TAKEDA CHEMICAL INDUSTRIES LTD) * page 10, line 54 - page 11, line 9; claims 9-13; figures 1, 2 * | 1-6, 9 | C12N C07K C12P |
| A | EP-A-315118 (TAKEDA CHEMICAL INDUSTRIES,LTD.) * page 13, line 6 - page 13, line 39; figure 4 * | 1-7, 9 | |

The present search report has been drawn up for all claims

| Place of search | Date of completion of the search | Examiner |
|---|---|---|
| THE HAGUE | 17 JANUARY 1991 | LE CORNEC N.D.R. |